(19) Europäisches Patentamt
European Patent Office
Office européen des brevets

(11) Publication number: **0 329 904 A1**

(12) **EUROPEAN PATENT APPLICATION**

(21) Application number: 88400417.7

(51) Int. Cl.4: A61K 31/435

(22) Date of filing: 23.02.88

(43) Date of publication of application: 30.08.89 Bulletin 89/35

(84) Designated Contracting States: FR

(71) Applicant: MERRELL DOW PHARMACEUTICALS INC.
P.O. Box 156300 2110 East Galbraith Road
Cincinnati Ohio 45215-6300(US)

(72) Inventor: Gittos, Maurice W.
16, rue André Malraux
F-67115 Plobsheim(FR)
Inventor: Moser, Paul
20, rue du Chanoine Straub
F-67100 Strasbourg(FR)

(74) Representative: Gillard, Marie-Louise et al
Cabinet Beau de Loménie 55, Rue d'Amsterdam
F-75008 Paris(FR)

# (54) Use of quinolizine and quinolizinone derivatives in the manufacture of medicaments for the treatment of anxiety.

(57) The present invention is directed to the use of esters of hexahydro-8-hydroxy-2,6-methano-2H-quinolizin-3-(4H)-ones and hexahydro-8-hydroxy-2,6-methano-2H-quinolizines in the manufacture of anxiolytic agents.

EP 0 329 904 A1

Xerox Copy Centre

# Use of quinolizine and quinolizinone derivatives in the manufacture of medioaments for the treatment of anxiety

The present invention is directed to the use of quinolizine and quinolizinone derivatives in the manufacture of a medicament for the treatment of anxiety.

In accordance with the present invention it has been discovered that anxiety can be treated, in a patient, in need thereof, by the administration of an anxiolytic quantity of a compound of the formula:

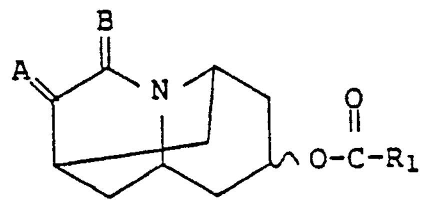

Formula I

wherein A is $H_2$, O, (H)(OH), $(OH)_2$ or N-OH; B is $H_2$, $(H)(CH_3)$, $(H)(CH_2NR_3R_4)$ or $CH_2$ wherein $R_3$ and $R_4$ are $C_{2-4}$ alkyl or are combined to give tetramethylene, pentamethylene or $-CH_2CH_2-0-CH_2CH_2-$; $R_1$ is

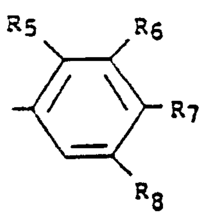 , 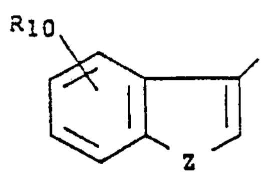 , 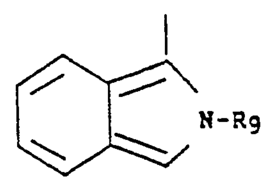 or 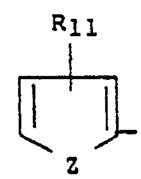

wherein Z is $NR_9$, O or S; $R_5$, $R_6$ and $R_8$ are each hydrogen, halogen, $C_{1-3}$ alkyl or $C_{1-3}$ alkoxy; $R_7$ is hydrogen, amino, ($C_{1-4}$ alkyl)amino, ($C_{1-4}$ alkyl)$_2$amino, alkoxy or nitro; $R_9$ is hydrogen, $C_{1-4}$ alkyl or phenyl ($C_{1-2}$ alkyl); $R_{10}$ is hydrogen, halogen, $C_{1-4}$ alkyl, $C_{1-4}$ alkoxy, hydroxy, cyano or $-CONH_2$; $R_{11}$ is hydrogen, halogen, $C_{1-4}$ alkyl or phenyl; the wavy line indicates that the configuration of the oxygen substituent on the ring can be endo or exo; and the pharmaceutically acceptable acid addition and quaternary ammonium salts of the aforesaid compounds.

Examples of the $C_{1-4}$ alkyl groups referred to above are methyl, ethyl, propyl, isopropyl and butyl. Examples of the $C_{1-4}$ alkoxy groups are methoxy, ethoxy, propoxy and butoxy. The halogens referred to above can be fluorine, chlorine or bromine. When the wavy line in the general structural formula is changed to a solid line, this indicates that the configuration of the compounds is endo. Such endo-compounds can also be referred to as trans. Similarly, exo-compounds can also be referred to as cis. Any hydrates of the present compounds are considered as equivalent to the compounds themselves and this would include compounds in which the carbonyl (i.e., A is O) exists as $(OH)_2$.

As used in this application, the term "anxiety" refers to a condition where a patient is experiencing fear, apprehension, uncertainity, etc.,and can be accompanied with physical manifestations such as, tachycardia, tremors, sweating, etc.

The term "patient" as used herein is taken to mean warm-blooded animals, such as mammals, for example, dogs, rats, mice, cats, guinea pigs, horses, cattle, sheep and primates, including humans.

A preferred group of compounds for use in the present invention are those wherein the ester is attached to the polycyclic ring in the endo-configuration. A further preferred group are those having the endo-configuration wherein A is O and $(OH)_2$. In a still further preferred group, B is additionally $H_2$.

The pharmaceutically acceptable acid addition salts referred to above can be non-toxic salts with suitable acids such as those with inorganic acids, for example hydrochloric, hydrobromic, nitric, sulfuric or phosphoric acids; or with organic acids such as organic carboxylic acids, for example, acetic, propionic, glycolic, maleic, hydroxymaleic, malic, tartaric, citric, salicylic, 2-acetyloxybenzoic, nicotinic or isonicotinic,; or organic sulfonic acids, for example methanesulfonic, ethanesulfonic, 2-hydroxyethanesulfonic, 4-toluenesulfonic or 2-naphthalensulfonic. Quaternary ammonium salts are formed with alkyl halides such as

methyl chloride, methyl bromide or ethyl bromide; or with sulfate esters such as methyl 4-toluenesulfonate or methyl 2-naphthalenesulfonate.

Some specific examples of compounds encompassed by the present invention are the following:

endo-8-(3,5-Dimethylbenzoyloxy)hexahydro-2,6-methano-2H-quinolizin-3(4H)-one
exo-8-(3,5-Dimethylbenzoyloxy)hexahydro-2,6-methano-2H-quinolizin-3(4H)-one
endo-8-(3,5-Dichlorobenzoyloxy)hexahydro-2,6-methano-2H-quinolizin-3(4H)-one
endo-8-(3,5-Dimethoxybenzoyloxy)hexahydro-2,6-methano-2H-quinolizin-3(4H)-one
endo-8-(4-Aminobenzoyloxy)hexahydro-2,6-methano-2H-quinolizin-3(4H)-one
endo-8-(4-Dimethylaminobenzoyloxy)hexahydro-2,6-methano-2H-quinolizin-3(4H)-one
endo-8-(3,5-Dimethylbenzoyloxy)octahydro-2,6-methano-2H-quinolizine
endo-8-(3-Indolylcarbonyloxy)octahydro-2,6-methano-2H-quinolizine
endo-8-(5-Cyano-3-indolylcarbonyloxy)hexahydro-2,6-methano-2H-quinolizin-3(4H)-one
endo-8-(3,5-Dichlorobenzoyloxy)hexahydro-2,6-methano-4-methyl-2H-quinolizin-3(4H)-one
endo-8-(3-Indolylcarbonyloxy)hexahydro-4-(diethylaminomethyl)-2,6-methano-2H-quinolizin-3(4H)-one
endo-8-(3-Indolylcarbonyloxy)octahydro-3-hydroxyimino-2,6-methano-2H-quinolizine
endo-8-(2-Methyl-1-isoindolylcarbonyloxy)hexahydro-2,6-methano-2H-quinolizin-3(4H)-one
endo-8-(2-Pyrrolidinylcarbonyloxy)hexahydro-2,6-methano-2H-quinolizin-3(4H)-one
endo-8-(3-Indolylcarbonyloxy)octahydro-2,6-methano-2H-quinolizin-3-ol
endo-8-(3-Chloro-5-methylbenzoyloxy)hexahydro-2,6-methano-2H-quinolizin-3(4H)-one

The compounds of the present invention can be prepared by reacting an alcohol or a reactive derivatives thereof, said alcohol having the formula:

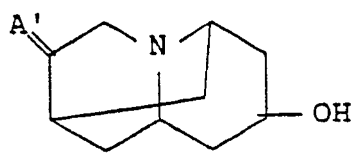

wherein A′ is O or $H_2$, with a reactive equivalent of an acid of the formula:

$R_1COOH$

wherein $R_1$ is defined as above. By a reactive equivalent of the acid is meant the corresponding acid chloride or bromide or the corresponding glyoxylyl chloride or bromide or the carboxylic acid imidazole obtained by the reaction of the appropriate acid halide with N,N-carbonyldiimidazole; or any similar acid derivative which would yield the simple carboxylic acid ester on reaction with an alcohol or with a reactive derivative of an alcohol. More specifically, where the -OH in the alcohol is equatorial (exo), then it can be reacted with the appropriate carboxylic acid imidazole obtained by the reaction of the acid halide with N,N-carbonyldiimidazole. Alternatively, the acid can be converted to the acid chloride by standard procedures (e.g., thionyl chloride) and then reacted with the alcohol or an alkali metal salt of the alcohol such as the lithium salt obtained by the reaction of lithium hydride with the alcohol in tetrahydrofuran.

When the -OH group in the starting alcohol is axial (endo), it can also be converted to the corresponding ester by reaction with the appropriate acid chloride or bromide with the reaction being carried out in the presence of an equivalent of a suitable tertiary base such as 4-dimethylaminopyridine in a high boiling inert solvent such as xylene. In this case, however, long heating (24-84 hours) at a temperature at or above 140°C is necessary so that the procedure would not be suitable for use with acid halides that are not stable under the indicated conditions. Thus, it was necessary to use an alternative for the preparation of such compounds. In this procedure, an appropriate acid chloride or bromide or a glyoxylyl chloride or bromide, in a nitroparaffin solvent, is reacted with a solution of a super acid salt of the alcohol and an equivalent amount of a heavy metal salt of the same super acid. The glyoxylyl chloride can be used in the process as indicated because it decarbonylates readily under the conditions used. The reaction itself can be carried out over a period of 1-24 hours at temperatures ranging from -80°C to ambient temperatures (about 23°C). Examples of suitable super acids with M = H are $MBF_4$, $MAsF_6$, $MSbF_6$, $MPF_6$, $MTaF_6$ or $MNbF_6$ with examples of suitable heavy metals (M) being silver and thallium. Examples of nitroparaffin solvents are nitromethane, nitroethane, 1-nitropropane and 2-nitropropane.

Actually, where the group $R_1$ contains a primary or secondary amino group, it is usually protected during the above reaction, with a benzyl group being commonly used to protect a secondary amine and a benzyloxycarbonyl group being used to protect a primary amine. In either case, the protecting group in the product is removed by conventional procedures, for example by hydrogenation with hydrogen and a

palladium catalyst.

Various procedures can be used to convert those compounds wherein A is O and whose preparation is described below, to other different bridged derivatives of the present invention by standard methods. Thus, the ketone group in the polycyclic system can be reduced to the corresponding alcohol using an alkali metal (sodium or potassium) borohydride in a lower alkanol such as methanol or ethanol.

The ketone group can also be reduced completely to a methylene group by a two step procedure. In the first step, the ketone is reacted with ethylene dithiol or trimethylene dithiol in the presence of a strong acid such as hydrochloric acid or $BF_3$ to give the corresponding dithioketal. The reaction is carried out in a suitable polar solvent such as nitromethane or acetic acid. The dithioketal is then reduced with hydrazine in the presence of Raney nickel in a lower alkanol solvent such a 2-propanol at elevated temperatures (60-100°C). Actually this same procedure can be used to reduce the original starting alcohol, hexahydro-8-hydroxy-2,6-methano-2H-quinolizin-3(4H)-one, to 8-hydroxy-2,6-methanooctahydro-2H-quinolizine which can itself be reacted with acid derivatives as described earlier to give the corresponding esters.

Compounds containing other B-groups (i.e. aminomethyl, methylene or methyl groups) can be obtained from products in which A is O and B is $H_2$ by a Mannich reactions using formaldehyde and a secondary amine such as dimethylamine, diethylamine, piperidine or pyrrolidine. This reaction gives the corresponding aminomethyl compound and, when B is dimethylaminomethyl, the amino moiety is eliminated on heating at 90-110°C in an inert solvent such as toluene to give the corresponding methylene compound (B is $CH_2$). This exocyclic methylene compound can be isolated by standard methods and transformed into a methyl group by hydrogenation, for example, by using hydrogen and platinum oxide.

To obtain those compounds in which A is hydroxyimino (N-OH), the ketone referred to above can be reacted with hydroxylamine hydrochloride by standard procedures.

The alcohol used as a reactant in the above procedure can be obtained from known alkyl ($C_{1-4}$) 3-cyclopentene-1-carboxylates by a multi-step procedure. Specifically, the double bond in the indicated cyclopentene is oxidized to a 1,2-diol using N-methyl-morpholine N-oxide in the presence of osmium tetroxide catalyst. The diol is then cleaved to the corresponding dialdehyde using sodium metaperiodate. A Robinson-Schöpf cyclization of the dialdehyde with a lower alkyl glycine ester and acetone-dicarboxylic acid, preferably at $pH_4$, gives a pseudopelletierine derivative of the following type:

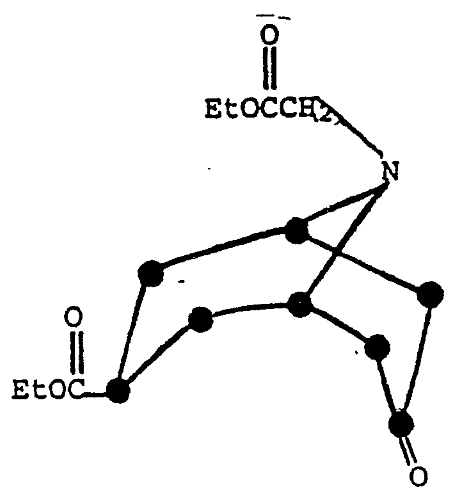

The ketone group is reduced to an alcohol using sodium borohydride and the product is reacted with dihydropyran to protect the -OH group as a tetrahydropyranyl ether. Dieckmann cyclization of the diester using a strong base (e.g. potassium t-butoxide) followed by aqueous acid hydrolysis and decarboxylation gives the desired alcohol. The resulting alcohols can exist in two conformations -axial and equatorial. The main product obtained by the above procedure is the axial alcohol and it can be separated from the equatorial isomer by crystallization of the camphorsulfonate or tetrafluoroborate salt.

The compounds represented by Formula I are 5-HT M-receptor antagonists and are useful the the treatment of anxiety, and in the manufacture of a medicament, therefor.

The activity of the compounds against 5-HT can be assessed by determining their $pA_2$ values in the isolated rabbit heart as described by J.R. Fozard et al., Eur. J. Pharmacol., 59, 195-210 (1979). In the method described, the molar concentration of antagonist which reduces the effects of twice the $ED_{50}$ of 5-HT to that of the $ED_{50}$ in the absence of antagonist is determined. The $pA_2$ value is the negative logarithm

of said molar concentrations. In general terms, the higher the $pA_2$ value the more potent is the compound. When tested in this way, the present compounds show $pA_2$'s generally in the range of about 8 to 10.

The activity of these compounds against 5-HT can be assessed in vivo by measurement of the effect of the compound on the Von Bezold-Jarisch Reflex induced by 5-HT injected intravenously into the rat (see Paintal A.S., Physiol. Rev. 53, 159-227, 1973; J.R. Fozard, Naunyn-Schmiedeberg's Arch. Pharmacol., 326, 1984, 36-44). The transient cardiac slowing arises from an increased afferent vagus activity arising from stimulation by 5-HT of sensory afferent fibers in and around the heart. When tested against the Von Bezold-Jarisch Reflex induced by 5-HT, compounds endo-8-(3,5-dimethylbenzoyloxy)hexahydro-2,6-methano-2H-quinolizin-3(4H)-one hydrochloride and endo-hexahydro-8-(3-indolylcarbonyloxy)-2,6-methano-2H-quinolizin-3(4H)one hydrochloride suppressed the response dose-dependently at doses of 0.01-0.1 mg/kg given intravenously or 0.25-1 mg/kg given orally.

The present compounds appear to be highly selective in their action against the 5-HT M-receptor. Their potency against other 5-HT receptors and other spasmogens, in particular carbachol, phenylephrine, histamine and calcium, is known to be at least three orders lower that that against 5-HT M-receptors.

The compounds of Formula I are useful in the treatment of anxiety; that is relieving or alleviating the apprehension, fear, or uncertainty, etc., that patients suffering from anxiety commonly experience, as well as relieving or alleviating the physiological changes associated with anxiety such as tachycardia, tremors, sweating, etc.

The compounds of Formula I possess a significant advantage over the anxiolytic agents which are currently available to clinicians, such as chlordiazepoxide, diazepam, and other benzodiazepines. The benzodiazepines commonly cause sedation and impairment of motor skills at the dosage levels commonly used in the treatment of anxiety.

The compounds of Formula I do not suffer from this disadvantage. They exhibit a wide dosage range at which they demonstrate anxiolytic activity, without causing either sedation or impairment of motor skills.

The quantity of compound required to produce the anxiolytic effect described above will vary with the particular compound utilized, the patient, the route of administration, the severity of the patient's anxiety, the presence of other underlying disease states in the patient, and other medications which are being administered concurrently to the patient. Generally though, a patients anxiety will respond to dosage range of from 0.01 to 10 mg/kg/day.

For oral administration, the compounds can be formulated into solid or liquid preparations such as capsules, pills, tablets, lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release medicaments. In another embodiment, the compounds of Formula I can be tableted with conventional tablet bases such as lactose, sucrose, and cornstarch in combination with binders, such as acacia, cornstarch, or gelatin, disintegrating agents such as potato starch or algenic acid, and a lubricant such as stearic acid or magnesium stearate. Liquid preparations are prepared by dissolving the active ingredient in an aqueous or non-aqueous pharmaceutically acceptable solvent which may also contain suspending agents, sweetening agents, flavoring agents, and preservative agents as are known in the art.

For parenteral administration, the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc. as are known in the art.

The following examples are presented in order to further illustrate the compounds used in the present invention. However, they should not be construed as limiting the scope of the invention in any manner. These compounds are disclosed and claimed in European Patent Application 87116119.6.

## EXAMPLE I

To a stirred solution of 160 g of diethyl malonate in 1.5 l of dry dimethylformamide at 0° C under nitrogen was slowly added 30 g of lithium hydride. After the evolution of hydrogen ceased (2 hours) 143 g of cis-1,4-dichloro-2-butene was slowly added and the mixture allowed to come to room temperature. After 72 hours, the mixture was diluted with a mixture of ether and hexane (1:4) and poured into water. The organic layer was washed with water and brine before drying over magnesium sulfate. Distillation gave diethyl 3-cyclopentene-1,1-dicarboxylate, bp 70-80° C/0.1 mm, containing a small amount (~10%) of diethyl

2-vinylcyclopropane-1,1-dicarboxylate.

The impure cyclopentene diester (148.5 g) obtained above was added to a solution of 118 g of potassium hydroxide in 1333 ml of 80% ethanol and the stirred solution warmed at 60-70° C overnight. The ethanol was evaporated and the residue treated with an ice cold solution of concentrated sulphuric acid (107 ml) in water (274 ml). Extraction of the acid mixture with ether (3 x 400 ml) followed by evaporation of the dried ether extracts gave a residue of the diacid which was decarboxylated to the monoacid by heating in an oil bath at 170-180° C for 1 hour. The residual oil was distilled to give crude 3-cyclopentene-1-carboxylic acid, bp 68-73° C (1 mm) containing some γ-vinyl-γ-butyrolactone. A solution of 98 g of potassium carbonate in 300 ml of water was added and the mixture extracted with ether to remove the γ-vinyl-γ-butyrolactone. Acidification of the aqueous solution and extraction with ether afforded pure 3-cyclopentene-1-carboxylic acid.

## EXAMPLE II

A mixture of 52 g of 3-cyclopentene-1-carboxylic acid and excess thionyl chloride was stirred at room temperature for 1 hour. The excess thionyl chloride evaporated and the residue distilled to give 3-cyclopentene-1-carbonyl chloride, bp 52-58° C.

The acid chloride obtained above was slowly added to an ice cooled stirred solution of 32 g of pyridine in 150 ml of ethanol. The mixture was stirred for a further hour, the ethanol evaporated and the residue treated with water and ether. The ether layer was separated, washed several times with water and dried. Evaporation of the ether left a residue of ethyl 3-cyclopentene-1-carboxylate, bp 62.5-66° C/14 mm.

## EXAMPLE III

A solution containing 84.6 g of N-methylmorpholine N-oxide, 1 g of osmium tetroxide, 230 ml of water and 115 ml of acetone was allowed to stir for 30 minutes at room temperature. To this stirred mixture was added very slowly over at least 8 hours, a solution of 80 g of ethyl 3-cyclopentene-1-carboxylate in 115 ml of acetone. The stirred mixture was heated at 50° C for 2 hours to complete the reaction (verified by TLC examination using ethyl acetate/hexane 70/30). Sodium bisulfite (~10 g) was added, the stirring continued for a further 15 minutes, and the mixture filtered through Celite. The pH of the filtrate was adjusted to 7 by the addition of 12 N sulfuric acid (37 ml), the acetone evaporated, the pH of the residual solution adjusted to 2 with 12 N sulfuric acid (13 ml) and the solution extracted with ethyl acetate (4 x 250 ml). Evaporation of the dried ethyl acetate solution gave 4-ethoxycarbonyl-1,2-cyclopentanediol.

## EXAMPLE IV

A solution of 85.4 g of sodium periodate in 500 ml of water was slowly added to a stirred solution of 69 g of 4-ethoxycarbonyl-1,2-cyclopentanediol in 690 ml of tetrahydrofuran. The reaction was exothermic and cooling was necessary. After two hours a precipitate of sodium iodate was filtered off and the solution concentrated at room temperature to remove most of the tetrahydrofuran. The resulting aqueous solution contained the desired β-ethoxycarbonylglutaraldehyde and was used directly in the next reaction.

To a stirred suspension of 400 g of potassium hydrogen phthalate in 800 ml of water was added, in sequence, a solution of 80 g of acetonedicarboxylic acid in 1200 ml of water, a solution of 80 g of glycine ethyl ester hydrochloride in 400 ml of water, and finally the solution of β-ethoxycarbonylglutaraldehyde obtained above. The mixture was stirred for 20 hours at room temperature during which time carbon dioxide evolved. The mixture was basified by the addition of an excess of aqueous potassium carbonate and extracted with ethyl acetate several times. Evaporation of the dried ethyl acetate extracts gave a syrup consisting mainly of 7-ethoxycarbonyl-9-(ethoxycarbonylmethyl)-9-azabicyclo-[3.3.1]nonan-3-one.

## EXAMPLE V

Sodium borohydride (17 g) was added in small portions to a stirred solution of 87.6 g of 7-ethoxycarbonyl-9-(ethoxycarbonylmethyl)-9-azabicyclo[3.3.1]nonan-3-one in 750 ml of ethanol. The mixture was stirred overnight at room temperature, the ethanol evaporated and the residue treated with 200 ml of water. Hydrochloric acid (2 M) was added until the mixture was acid and this acid solution was immediately basified by the addition of saturated potassium carbonate solution. Extraction with ethyl acetate and evaporation of the dried extract gave a syrup which consisted mainly of 7-ethoxycarbonyl-9-(ethoxycarbonylmethyl)-9-azabicyclo[3.3.1]nonan-3-ol. The syrup can be purified by column chromatography using silica and elution with hexane-ethyl acetate (30:70).

## EXAMPLE VI

A solution of 26.1 g of the crude 7-ethoxycarbonyl-9-(ethoxycarbonylmethyl)-9-azabicyclo[3.3.1]nonan-3-ol in 250 ml of methylene chloride was treated with one equivalent of methanesulfonic acid (8.42 g). The methylene chloride solution was concentrated to about 35 ml, 9.5 ml of dihydropyran was added together with one drop of methansulfonic acid, and the mixture stirred for 3 hours at room temperature. The mixture was then poured into saturated potassium carbonate solution and the product separated by extraction with ethyl acetate.

Evaporation of the dried ethyl acetate extracts gave a syrup consisting mainly of the tetrahydropyranyl ether of 7-ethoxy-carbonyl-9-(ethoxycarbonylmethyl)-9-azabicyclo[3.3.1]nonan-3-ol. It can be purified by column chromatography using silica and elution with hexane-ethyl acetate (20:80), Rf 0.7.

## EXAMPLE VII

A solution of 34 g of the tetrahydropyranyl ether of 7-ethoxycarbonyl-9-(ethoxycarbonylmethyl)-9-azabicyclo[3.3.1]nonan-3-ol in 800 ml of anhydrous toluene was treated with 19 g of potassium tert-butoxide and the stirred mixture heated at 100° C for 2 hours. Anhydrous formic acid (7.85 g) was added to the cooled mixture, the potassium formate was filtered off, and the toluene solution evaporated to give a syrup. The syrup was treated with 300 ml of 5 N hydrochloric acid and the stirred solution refluxed overnight. The cooled mixture was clarified by an extraction with methylene chloride and the aqueous acid solution evaporated to dryness. The residue was dissolved in a little water and the solution treated with a large excess of saturated potassium carbonate solution. Extraction of the resulting mixture with ethyl acetate and evaporation of the dried ethyl acetate solution gave endo-hexahydro-8-hydroxy-2,6-methano-2H-quinolozin-3(4H)-one as an oil which crystallized on standing. The base was converted to its camphorsulfonate salt, m.p. 178° C, using one equivalent of camphorsulfonic acid in ethanol.

## EXAMPLE VIII

A mixture of 1.8 g of endo-hexahydro-8-hydroxy-2,6-methano-2H-quinolizin-3(4H)-one, hydrofluoroboric acid (0.88 g; 60% aqueous solution) and 20 ml of ethanol was evaporated, the residue was treated with 50 ml of anhydrous toluene, and the mixture again evaporated. A stirred suspension of the anhydrous residue in 50 ml of anhydrous nitroethane at -78° C was treated with 1.94 g of anhydrous silver tetrafluoroborate and a solution of 1.7 g of 3,5-dimethylbenzoyl chloride in 20 ml of anhydrous nitroethane was added slowly. The temperature of the stirred reaction was kept at -78° C for 1.5 hours and then allowed to return to room temperature overnight. Triethylamine (1 g) was added, the solution filtered and the nitroethane evaporated. A solution of the residue in 20 ml of water was treated with an excess of a saturated aqueous solution of potassium carbonate and the liberated oil separated by extraction with ethyl acetate. The ethyl acetate solution was washed several times with water before being dried over magnesium sulfate and evaporated. The residue obtained was endo-8-(3,5-dimethylbenzoyloxy)hexahydro-2,6-methano-2H-quinolizin-3(4H)-one and this was treated with methylene chloride and ethereal hydrogen chloride to give crystals of the hydrochloride salt melting at about 291° C.

## EXAMPLE IX

When the procedure of Example VIII is repeated using endohexahydro-8-hydroxy-2,6-methano-2H-quinolizin-3(4H)-one and the appropriate acid chloride, the corresponding esters listed below are obtained. As necessary, the acid chlorides were obtained from the appropriate carboxylic acids by standard procedures, for example, using thionyl chloride. To convert the ester to a corresponding acid salt, it was reacted with the appropriate acid with alternative solvents being used as desired.

endo-Hexahydro-8-(3-indolylcarbonyloxy)-2,6-methano-2H-quinolizin-3(4H)-one methansulfonate melting at about 278° C.
endo-8-(3-Benzofurancarbonyloxy)hexahydro-2,6-methano-2H-quinolizin-3(4H)-one
endo-8-(3-Benzo[b]thiophenecarbonyloxy)hexahydro-2,6-methano-2H-quinolizin-3(4H)-one
endo-8-(1-Benzyl-1H-indol-3-ylcarbonyloxy)hexahydro-2,6-methano-2H-quinolizin-3(4H)-one
endo-Hexahydro-8-(1-methyl-1H-indol-3ylcarbonyloxy)-2,6-methano-2H-quinolizin-3(4H)-one
endo-8-(4-Bromo-2-furylcarbonyloxy)hexahydro-2,6-methano-2H-quinolizin-3(4H)-one
endo-Hexahydro-8-(5-phenyl-2-furylcarbonyloxy)-2,6-methano-2H-quinolizin-3(4H)-one
endo-8-(3-Chloro-2-thienylcarbonyloxy)hexahydro-2,6-methano-2H-quinolizin-3(4H)-one
endo-Hexahydro-8-(5-methyl-2-thienylcarbonyloxy)-2,6-methano-2H-quinolizin-3(4H)-one
endo-Hexahydro-8-(1-methyl-1H-pyrrol-2-ylcarbonyloxy)-2,6-methano-2H-quinolizin-3(4H)-one
endo-8-(3-Chloro-4-nitrobenzoyloxy)hexahydro-2,6-methano-2H-quinolizin-3(4H)-one
endo-8-(3-Chloro-4-dimethylaminobenzoyloxy)hexahydro-2,6-methano-2H-quinolizin-3(4H)-one
endo-8-(3,5-Dichlorobenzoyloxy)hexahydro-2,6-methano-2H-quinolizin-3(4H)-one
endo-8-(3,5-Dimethoxybenzoyloxy)hexahydro-2,6-methano-2H-quinolizin-3(4H)-one
endo-8-(2,5-Dimethylbenzoyloxy)hexahydro-2,6-methano-2H-quinolizin-3(4H)-one

## EXAMPLE X

Oxalyl chloride (0.76 ml) was slowly added to a stirred solution of 1 g of 5-methylindole in 20 ml of anhydrous ether at 0° c. The precipitate which formed was filtered off and dried at 80° C to give 5-methyl-3-indolylglyoxylyl chloride.

A stirred solution of 205 mg of anhydrous silver tetrafluoroborate in 10 ml of anhydrous nitroethane was treated with a solution of 282.5 mg of endo-hexahydro-8-hydroxy-2,6-methano-2H-quinolizin-3(4H)-one tetrafluoroborate (obtained by treating the free amine with an equivalent of hydrofluoroboric acid) in 10 ml of anhydrous nitroethane at room temperature. A solution of 233 mg of 5-methyl-3-indolylglyoxylyl chloride in 10 ml of anhydrous nitroethane was slowly added and the mixture stirred at room temperature overnight. Triethylamine (101 mg) was added, the solution filtered and the nitroethane evaporated. A solution of the residue in 15 ml of water was treated with a saturated aqueous solution of potassium carbonate and the liberated oil separated by extraction with ethyl acetate. The ethyl acetate solution was washed several times with water before being dried over magnesium sulfate and evaporated. The residue was treated with methylene chloride and ethereal hydrogen chloride, and the solid filtered off and recrystallized from 2-propanol to give endo-hexahydro-8-(5-methyl-3-indolylcarbonyloxy)-2,6-methano-2H-quinolizin-3(4H)-one hydrochloride.

When the above procedure was repeated using the appropriate substituted indole in place of the 5-methylindole, the following compounds were obtained:

endo-Hexahydro-8-(5-chloro-3-indolylcarbonyloxy)-2,6-methano-2H-quinolizin-3(4H)-one hydrochloride melting at about 317-320° C (with decomposition) after recrystallization from ethanol.
endo-Hexahydro-8-(5-cyano-3-indolylcarbonyloxy)-2,6-methano-2H-quinolizin-3(4H)-one hydrochloride melting at about 304-305° C (with decomposition) after recrystallization from ethanol.
endo-Hexahydro-8-(5-methoxy-3-indolylcarbonyloxy)-2,6- methano-2H-quinolizin-3(4H)-one hydrochloride melting at about 303° C (with decomposition) after recrystallization from isopropanol.

Also obtained in the same way are endo-Hexahydro-8-(5-carbamoyl-3-indolylcarbonyloxy)-2,6-methano-2H-quinolizin-3(4H)-one and endo-Hexahydro-8-(5-hydroxy-3-indolylcarbonyloxy)-2,6-methano-2H-quinolizin-3(4H)-one. In the later case, the starting material is 5-benzyloxyindole and the initial product is debenzylated by reduction using standard procedures.

EXAMPLES XI

Dimethylamine (40% solution in water, 0.68 g) and formaldehyde (30% solution in water, 0.49 g) were successively added to a solution of 1.25 g of endo-8-(3,5-dimethylbenzoyloxy)hexahydro-2,6-methano-2H-quinolizin-3(4H)-one in a mixture of 4 ml of ethanol and 2 ml of water. The stirred mixture was heated at 70-75° C for 16 hours and concentrated. Toluene (50 ml) was added and the mixture evaporated at 110° C.

A solution of the residue [which contained endo-8-(3,5-dimethylbenzoyloxy)hexahydro-4-methylene-2,6-methano-2H-quinolizin-3(4H)-one] in 30 ml of ethanol was hydrogenated at room temperature and atmospheric pressure in the presence of 0.2 g of platinum oxide (Adams catalyst). One equivalent of hydrogen was absorbed in one hour. The catalyst was filtered off, the ethanol evaporated and the residue treated with one equivalent of hydrofluoroboric acid in water. Evaporation of the aqueous solution gave a crystalline residue which was recrystallized from ethanol to give endo-8-(3,5-dimethylbenzoyloxy)hexahydro-4-methyl-2,6-methano-2H- quinolizin-3(4H)-one tetrafluoroborate melting at about 270-275° C.

EXAMPLE XII

A solution of endo-8-(3-indolylcarbonyloxy)hexahydro-2,6-methano-2H-quinolizin-3(4H)-one (1.42 g) in ethanol (5 ml) was treated with fluoboric acid (0.64 g, 60% aqueous solution) and the mixture evaporated to give endo-8-(3-indolylcarbonyloxy)-hexahydro-2,6-methano-2H-quinolizin-3(4H)-one tetrafluoroborate (1.8 g).

A stirred suspension of the above salt (1.8 g) in anhydrous nitroethane (30 ml) was treated with propane-1,3-dithiol (3 ml) and boron trifluoride etherate (3 drops) and the mixture stirred overnight at room temperature. The nitroethane was removed by evaporation and the residue triturated with ether. The solid product was filtered off, washed several times with ether, treated with water (25 ml), saturated aqueous potassium carbonate (3 ml) and ether (50 ml). The ether solution was separated off, dried ($MgSO_4$) and evaporated to give the propane dithioketal derivative, m.p. 226-229° C (1.6 g).

Hydrazine hydrate (3 ml) was added dropwise during one hour to a stirred refluxing solution of the above dithioketal (0.5 g) in isopropanol (20 ml) in the presence of Raney nickel (6 g, previously washed three times with isopropanol). The reflux was maintained for a further 30 minutes, the hot solution filtered through a triple superphosphate, the nickel washed several times with hot isopropanol and the combined filtrates evaporated to give endo-8-(3-indolylcarbonyloxy)octahydro-2,6-methano-2H-quinolizine as the free base (50 mg). Addition of methylene chloride and ethereal hydrogen chloride gave the hydrochloride (30 mg), m.p. 311-313° C (from ethanol).

EXAMPLE XIII

The procedure of Example XII was repeated using endo-hexahydro-8-hydroxy-2,6-methano-2H-quinolizin-3(4H)-one in place of the ester. The dithioketal obtained was reduced as described in the final paragraph except that the hydrazine hydrate was left out. This gave exo-octahydro-2,6-methano-2H-quinolizin-8-ol which was then reacted with 3,5-dimethylbenzoyl chloride to give exo-8-(3,5-dimethylbenzoyloxy)octahydro-2,6-methano-2H-quinolizine which was converted to the hydrochloride, m.p. 255-256° C by standard procedures.

**Claims**

1. The use in the manufacture of a medicament for the treatment of anxiety of compounds of the following general formula:

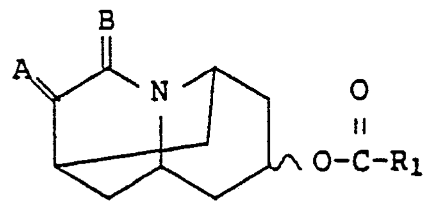

Formula I

wherein A is $H_2$, O, (H)(OH), $(OH)_2$ or N-OH; B is $H_2$, $(H)(CH_3)$, $(H)(CH_2NR_3R_4)$ or $CH_2$, wherein $R_3$ and $R_4$ are $C_{2-4}$ alkyl or are combined to give tetramethylene, pentamethylene or $-CH_2CH_2$-0-$CH_2CH_2$-; $R_1$ is

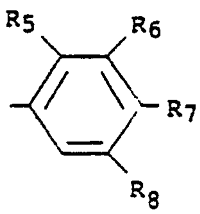 , 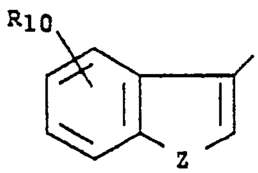 , 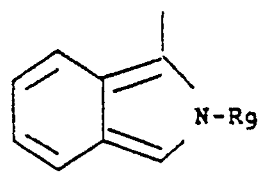 or 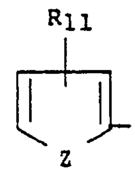

wherein Z is $NR_9$, O or S; $R_5$, $R_6$ and $R_8$ are each hydrogen, halogen, $C_{1-3}$ alkyl or $C_{1-3}$ alkoxy; $R_7$ is hydrogen, amino, ($C_{1-4}$ alkyl)amino, ($C_{1-4}$ alkyl)$_2$amino, alkoxy or nitro; $R_9$ is hydrogen, $C_{1-4}$ alkyl or phenyl ($C_{1-2}$ alkyl); $R_{10}$ is hydrogen, halogen, $C_{1-4}$ alkyl, $C_{1-4}$ alkoxy, hydroxy, cyano or $-CONH_2$; $R_{11}$ is hydrogen, halogen, $C_{1-4}$ alkyl or phenyl; the wavy line indicates that the configuration of the oxygen substituent on the ring can be endo or exo; and the pharmaceutically acceptable acid addition and quaternary ammonium salts of the aforesaid compounds.

European Patent Office

# EUROPEAN SEARCH REPORT

Application Number

EP 88 40 0417

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| T,D | EP-A-0 266 730 (MERREL DOW PHARMACEUTICALS INC.)<br>* Summary *<br>----- | 1 | A 61 K 31/435 |
| | | | TECHNICAL FIELDS SEARCHED (Int. Cl.4) |
| | | | A 61 K 31/00 |

The present search report has been drawn up for all claims

| Place of search | Date of completion of the search | Examiner |
|---|---|---|
| THE HAGUE | 16-09-1988 | THEUNS H.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)